# EUROPEAN PATENT SPECIFICATION

(11) **EP 1 129 352 B1**
(45) Date of publication and mention of the grant of the patent: **02.02.2005**
(21) Application number: 99954218.6
(22) Date of filing: 11.11.1999
(51) Int. Cl.: G01N 33/53, C12Q 1/42

(54) **ASSAY FOR PHOSPHATASE-TARGETING TOXINS**
TEST FÜR ZIELPHOSPHATASETOXINEN
DOSAGE DES TOXINES DE CIBLAGE DE PHOSPHATASES

(30) Priority: 11.11.1998 GB 9824772
(43) Date of publication of application: 05.09.2001
(73) Proprietor: Biosense Laboratories AS, 5008 Bergen (NO)
(72) Inventor: DOSKELAND, Stein, Ove, University of Bergen, 5009 Bergen (NO); SERRES, Margrethe, Hauge, University of Bergen, 5009 Bergen (NO); FLADMARK, Kari, Espolin, University of Bergen, 5009 Bergen (NO)
(74) Representative: Cockbain, Julian, Dr.
(86) International application number: PCT/GB1999/003756
(87) International publication number: WO 2000/028325

(56) References cited:
- EP-A- 0 311 456
- EP-A- 0 554 458
- US-A- 5 180 665
- US-A- 5 525 525
- DATABASE BIOSIS [Online] BIOSCIENCES INFORMATION SERVICE, PHILADELPHIA, PA, US YANG, MENGSU (1) ET AL: "Effects of microcystins on phosphorylase-a binding to phosphatase-2A: Kinetic analysis by surface plasmon resonance biosensor." retrieved from STN XP002130132 & BIOCHIMICA ET BIOPHYSICA ACTA, (MARCH 14, 1999) VOL. 1427, NO. 1, PP. 62-73. ,

## Description

The present invention relates to an assay method for the detection of phosphatase-targeting toxins typically produced by microalgae such as for example cyanobacteria and dinoflagellates.

Dinoflagellates are typically unicellular, photosynthetic, bi-flagellated algae. Some of the marine dinoflagellates (e.g. *Prorocentrum sp.* and *Dinophysis sp.*) produce phosphatase-targeting toxins such as okadaic acid and dinophysis toxin, which cause gastrointestinal problems if ingested by humans. Such algae can thus be problematic if they contaminate the habitats of shellfish for consumption.

Cyanobacteria, which are often referred to as blue-green algae, are also photosynthetic organisms which are principally aquatic and inhabit coastal waters, open sea and oceans, rivers, lakes and ground water but may also be terrestrial and found in leaf litter and soil.

Many species and strains of cyanobacteria, in particular *Microcystis sp*., *Aphanizomenon sp*., *Anabena sp., Nodularia sp.* and *Oscillatoria sp.,* produce toxins which if ingested by humans or other mammals, birds and even fish, can produce illness. Ingestion of such toxins occurs by two main routes, either by drinking contaminated water or by eating contaminated seafood.

Two particular types of toxins are produced by cyanobacteria and dinoflagellates. Neurotoxins, for example anatoxins and saxitoxins, cause paralysis in the victim and hence the condition often referred to as paralytic shellfish poisoning. Poisoning by such neurotoxins is rare but can prove to be fatal.

The other form of toxins inactivate protein phosphatase enzymes in the cells of the body by binding to the enzymes and affecting their ability to dephosphorylate protein substrates. These toxins are relatively common, and some (such as the dinoflagellate toxins okadaic acid and dinophysis toxin) can cause nausea, vomiting and diarrhoea and hence the condition often referred to as diarrhoetic shellfish poisoning. Some protein phosphatase-targeting toxins are tumour promoters and exposure to these toxins may lead to cancer. Others, such as the cyanobacterial toxins microcystin and nodularin are hepatotoxic and cause liver damage. The most prevalent of the phosphatase targeting toxins are microcystin, nodularin and okadaic acid.

The most common sources of dinoflagellate toxin poisoning are shellfish and fish liver, and the most common cause of cyanobacterial toxin poisoning is contaminated drinking and/or bathing water. Both cyanobacterial and dinoflagellate toxins may however be harboured in shellfish and in water. A particularly common source of algal toxin poisoning is mussels since they accumulate the toxins upon feeding on toxin-producing algae. Other shellfish, for example oysters, clams and scallops can also be affected.

Additionally, domestic water supplies, particularly if they originate from ground water, can become contaminated with cyanobacteria and thus provide a direct route for toxin ingestion.

There is some concern regarding consumption of algae and cyanobacteria as a high-protein health food and diet aid. There are no official guidelines for monitoring collected algae or cyanobacteria for contamination by toxin producing strains and the marketing of genera such as *Anabena* and *Aphanizomenon* is particularly worrying since a number of toxin producing strains may be found within them.

In addition to the short term discomfort, medical costs, commercial costs to the shellfish industry, loss of working hours etc. which result from exposure to algal toxins, as mentioned above the phosphatase targeting toxins microcystin and nodularin have been found to be tumour promoters and it is believed that repeated exposure to such toxins at the clinical or sub-clinical level, particularly in combination with a high intake of alcohol or smoking may result in cancer, especially of the liver.

Presently, a number of different methods exist for the detection and quantitation of phosphatase targeting toxins, from algae and cyanobacteria. One standard method involves grinding mussels or other potential sources of the phosphatase targeting toxins and injecting an extract of the ground mussel tissue into mice. The presence and level of phosphatase-targeting toxin contamination is then determined in relation to mouse survival (Stabell *et al*. (1992), Food. Chem. Toxicol. 30(2): 139-44). Clearly, this is a time consuming, crude and expensive method of assessing food safety and quality control.

Another method for determining diarrheal shellfish poisons (DSP) (EP-A-554458 of Iatron Laboratories Inc) involves the use of a first and second antibody to the toxin in a conventional sandwich assay.

Another method involves measuring the reduction in enzymic activity of exogenously added phosphatase thus detecting the presence of phosphatase targeting toxins in the shellfish. Again this involves grinding mussels or other shellfish tissue, releasing endogenous phosphatates which interfere with the added phosphatase, compromising the sensitivity and accuracy of the test (Sim and Mudge (1994) in Detection Methods for Cyanobacterial Toxins Eds. Codd, Jeffries, Keevil and Potter, Royal Society of Chemistry, and US-A-5180665 of Charles Holmes.

A great need exists therefore for a quick, sensitive, and inexpensive assay or method to allow the qualitative and/or quantitative determination of the presence of phosphatase-targeting toxins, in particular algal and cyanobacterial phosphatase-targeting toxins, in water, shellfish and/or edible products of algae or cyanobacteria. In particular, there is a need for an assay method which is simple enough to be performed on site by relatively non-skilled or non-skilled personnel, for example fishmongers or water sanitation personnel and requires no laboratory equipment or special facilities for its performance.

Thus, according to a first aspect, the present invention provides an assay method for determining phosphatase targeting toxins which inhibit protein phosphatases comprising contacting a solid support having an immobilized ligand thereon with:
(i) a sample suspected of being contaminated with toxin and
(ii) a non-immobilized ligand,
wherein said immobilized ligand is capable of binding to at least one of said toxins, to said non-immobilized ligand or to complexes of said toxin and said non-immobilized ligand, and said non-immobilized ligand is capable of binding to at least one of said immobilized ligand, to said toxin or to complexes of said toxin and said immobilized ligand whereby the proportion of said immobilized ligand bound by said toxin, said non-immobilized ligand or complexes of said toxin and said non-immobilized ligand is dependent on the toxin content of said sample and
wherein said immobilized ligand is capable of generating directly or indirectly a detectable signal when uncomplexed, when complexed by said toxin, when complexed by a complex of said toxin and said non-immobilized ligand or when complexed by said non-immobilized ligand or said non-immobilized ligand is capable of generating a directly or indirectly detectable signal when uncomplexed or when complexed,
separating a bound fraction from a non-bound fraction; and
directly or indirectly determining the non-immobilized ligand bound to the immobilized ligand (the bound fraction) or non-complexed in aqueous solution (the non-bound fraction);
wherein the application of (i) and (ii) to the solid support may be performed separately, sequentially or simultaneously and if separately or sequentially, they can be performed in either order.

Thus in one embodiment toxin determination may involve determination of the non-immobilized ligand which has failed to bind directly or indirectly to the immobilized ligand. Where the non-immobilized ligand competes for binding to the immobilized ligand with the toxin a high level of unbound ligand is indicative of a high toxin concentration. Where the non-immobilized ligand can complex toxin bound to the immobilized ligand then a high level of unbound ligand is indicative of a low level of toxin concentration.

In another embodiment, toxin determination involves determination of the non-immobilized ligand which has bound directly or indirectly to the immobilized ligand. Where toxin and non-immobilized ligand compete for binding to the immobilized ligand then a high level of bound ligand is indicative of a low level of toxin concentration. Where the non-immobilized ligand can complex toxin bound to the immobilized ligand then a high level of bound ligand is indicative of a high level of toxin concentration.

Preferably however the method of the invention involves a competitive binding assay for the detection of phosphatase-targeting toxins, in particular algal and cyanobacterial toxins, wherein toxin molecules present in a sample compete with the non-immobilized ligand for a limited number of binding sites of the immobilized ligand and any toxin present in said sample is determined relative to the extent of non-immobilized ligand bound to or not bound to the binding sites of the immobilized ligand.

As used herein, the terms "detecting" "determining" or "assessing" include both quantitation in the sense of obtaining an absolute value for the amount or concentration of phosphatase-targeting toxins, present in the sample and also semi-quantitative and qualitative assessment or determination. An index, ratio, percentage or molar indication of the level or amount of toxin present may be determined or alternatively a simple indication of presence or absence of such toxins in the sample, may be obtained. In a preferred aspect of the invention a simple presence or absence or semi-quantitative determination of toxin presence is achieved. In this regard "absence" of toxin may mean that the toxin concentration is below the detection limit of the assay or is below a level deemed to be safe or tolerable.

The samples used in the assay method of the invention may be any sample suspected of exposure to phosphatase-targeting toxins, perhaps by exposure to phosphatase-targeting toxin producing microorganisms, for example water which may be sea water, fresh water, ground water, water taken from lakes, rivers, wells, streams, reservoirs, domestic water supplies or may be moisture extracted from shellfish for example by simple draining or extraction using a pipette or water in which shellfish have been allowed to soak or may be a foodstuff, food additive, nutritional supplement, alternative remedy or similar product which is produced by or from algae or cyanobacteria. Where shellfish contain free water (e.g. as in oysters), the assay may involve dipping an absorbent substrate (the solid support) into that water. Alternatively it may simply involve pressing an absorbent substrate against the damp flesh of the shellfish, e.g. after breaking on opening the shell.

In a preferred aspect of the invention the sample under investigation is surface or free moisture from shellfish.

All types of shellfish, for example scallops, prawns, mussels, and oysters are susceptible to the assay method of the invention but in a preferred aspect, the shellfish are mussels. In another preferred aspect, the sample under investigation is water taken from the habitat in which such shellfish live and in a further preferred aspect, the sample is water taken from domestic water supplies.

The sample used for analysis may be used in an essentially untreated manner but may optionally be filtered by any known method or diluted by adding water, buffer or any other aqueous medium prior to analysis and may be stored or preserved for example by chilling or freezing prior to analysis.

Any toxin binding ligand may be used in the method of the invention as the immobilized or non-immobilized ligand for example antibodies, which may be polyclonal or monoclonal, or antibody fragments for example F(ab), F(ab')₂ or F(v) fragments. Such antibodies or antibody fragments may be monovalent or divalent and may be produced by hybridoma technology or be of synthetic origin, either as products of recombinant DNA technology or chemical synthesis. Single chain antibodies or other antibody derivatives or mimics could for example be used. The antibodies or antibody fragments may be directed or raised against any epitope, component or structure of the phosphatase-targeting toxins as appropriate. Alternatively, compounds with an affinity for the toxin for example a small organic molecule or peptide, e.g. an oligopeptide or polypeptide, capable of specifically binding the toxin for example a specific binder selected from a combinatorial chemistry or phage display library or a specifically binding sequence of DNA or RNA could be used.

Preferably however, the toxin binding ligand of the present invention is a protein phosphatase enzyme, and even more preferably the binding ligand protein phosphatase 2A (pp2A) is used in the assay method.

Likewise, the second ligand used in the method of the invention may be any ligand which binds to the toxin either competitively or non competitively with the first ligand. Alternatively, the second ligand may be any ligand which competes with the toxin for binding to the first ligand. Preferably the first ligand is a toxin binding ligand, more preferably a protein phosphatase enzyme. One of the two ligands must be immobilized and the other must be non-immobilized and one of the ligands must be directly or indirectly detectable. In a preferred embodiment the non-immobilized ligand should meet the functional requirements that it competitively inhibits toxin binding to the immobilized ligand and can directly or indirectly produce a detectable signal, e.g. it may be a molecule which can be labelled using a direct or indirect signal forming moiety of any known form. Such ligands may likewise take the form of antibodies, which may be polyclonal or monoclonal, or antibody fragments for example F(ab), F(ab')₂ or F(b) fragments. Such antibodies or antibody fragments may be monovalent or divalent and may be produced by hybridoma technology or be of synthetic origin, either recombinant DNA technology or chemical synthesis. Single chain antibodies or other antibody derivatives or mimics and small organic molecules, peptides, oligopeptides and polypeptides selected from combinatorial or phage display libraries, could for example be used. The antibodies or antibody fragments may be directed or raised against any epitope, component or structure of the phosphatase-targeting toxin molecule or the ligand which binds the phosphatase targeting molecule as appropriate. Alternatively, compounds with an affinity for the toxin or for the ligand which binds the toxin, for example a small organic molecule or peptide, oligopeptide or polypeptide capable of specifically binding the toxin or the ligand which binds the toxin, for example a specific binder selected from a combinatorial chemistry or phage display library, or a specifically binding sequence of DNA or RNA could be used.

The reporter moiety which one of the ligands will generally carry may be a binding site for a directly detectable moiety, e.g. a metal sol (e.g. gold sol), a chromosphore or fluorophore (e.g. a cyanine, phthalocyanine, merocyanint, triphenylmethyl, equinance, etc. see Topics in Applied Chemistry, Infrared Absorbing Chromophores, edited by M. Matsuoka, Plenum Press, New York, NY, 1990, Topics in Applied Chemistry, The Chemistry and Application of Dyes, Waring et al. Plenum Press, New York, NY, 1990, and Handbook of Fluorescent Probes and Research Chemicals, Haugland, Molecular Probes Inc. 1996, a radiolabel, an enzyme, a magnetic particle, a turbidity inducing agent, etc., or it may already carry such a directly detectable moiety. Where the reporter moiety is carried by the immobilized ligand it will generally be a binding site for a directly detectable moiety which binding site is either activated, or more generally deactivated, when the ligand is complexed.

Preferably the reporter moiety is carried by the non-immobilized ligand.

In a preferred embodiment of the invention, the non-immobilized ligand is a labelled, e.g. enzyme or choromophore or fluorophore labelled peptide hepatotoxin, e.g. a hepatotoxin selected from nodularin, microcystin LR or microcystin YR or alternatively okadaic acid.

While labelling with radiolabels is possible, since the assay is primarily intended for on-site use by lay users, it is preferable to use reporter moieties that give a visible signal, e.g. chromophores, fluorophores, phosphorescent moieties, turbidity inducing agents, gas evolution inducing agents, etc.

Where the signal forming moiety is a material which binds to a binding site on one of the ligands, it will conveniently be contacted with the bound or unbound fraction, as appropriate, after separation of the bound and unbound fractions.

In general, where the signal is to be derived from the bound fraction, it will be preferable to rinse the substrate, e.g. with water, to flush away the unbound fraction before the ligand is detected or generated and detected.

Any species or strain of algae or cyanobacteria which produces phosphatase-targeting toxins may be subject to the present invention but it is particularly applicable to toxin producing strains of cyanobacteria for example *Microcystis aeroginosa, Anabena* species, *Nodularia spuragena* and *Anabena flus-aquae* or algae. Thus for example the toxins microcystin-LR and microcystin-YR are produced by *Microcystis sp.,* the toxin nodularin is produced by *Nodularia sp.* and the toxin okadaic acid is produced by *Prorocentrum sp.*

The toxins subject to determination by the present method may likewise be any phosphatase-targeting toxin produced by algae or cyanobacteria, but in preferred aspects the peptide toxins are hepatotoxins (of which microcystin and nodularin are the most prevalent) or okadaic acid.

Thus, in its most general sense, the method of the invention involves simply contacting a sample suspected of contamination with phosphatase-targeting toxins, with a toxin binding ligand and a reporter molecule capable of competing with said toxin for the binding sites of the ligands either simultaneously, sequentially or separately in either order, the reporter molecule optionally being bound to the binding ligand prior to exposure to the sample under investigation, and determining the reporter molecule which is either bound to the solid phase or free in solution.

The bound faction may be separated from the unbound faction prior to assessment of reporter by any suitable means, for example, precipitation, centrifugation, filtration, chromatographic means, capilliary action or simply by draining. The solid phase may for example be in the form of a dipstick or a solid matrix in any known form for example polymeric or magnetic beads for example Dynabeads® (available from Dynal AS). In preferred embodiments of the present invention, the solid phase to which the toxin binding ligands are immobilised is in the form of Dynabeads®.

The reporter molecule may be assessed in either the bound or the non-bound faction depending on the specific embodiment of the invention but preferably it is assessed in the bound fraction.

The immobilized ligand may be immobilised by any known means, for example by binding or coupling the ligand to any of the well known solid supports or matrices which are currently widely used or proposed for separation or immobilisation for example solid phases may take the form of particles, sheets, gels, filters, membranes, fibres or capillaries or microtitre strips, tubes or plates of wells etc. and conveniently may be made of glass, silica, latex, a polymeric material or magnetic beads. Techniques for binding the ligand to the solid support are well known in the art and widely described in the literature. In preferred embodiments of the present invention, the solid phase to which the phosphatase-targeting toxin binding ligands are immobilised is in the form of Dynabeads®.

The assay method of the present invention is advantageous in that it can be performed without the need of complex laboratory equipment and can be performed by the relatively non-skilled or non-skilled person. Hence, the assay method is suitable for use in the home, in shops or in the field and it can be performed quickly and easily without the need for intensive labour or hazardous chemicals.

Of particular advantage in the assay of the present invention is the very high degree of sensitivity which is of critical importance when analysing samples wherein the toxin is present at very low levels for example in the testing of drinking water or assessing possible pollution with phosphatase targeting toxins. Typically the assay is capable of detecting toxins in picomolar concentrations, e.g. as low as 10 pM. Conveniently the assay may be used to detect toxins in the 15 to 560 pM range.

A further advantage of the present assay relative to existing techniques is that the present assay is not affected by the presence of endogenous phosphatases which may be present in the samples under analysis, particularly, for example, if the samples are taken from shellfish.

In one embodiment of the present invention, a protein phosphatase is immobilised on a solid support, the immobilised phosphatase is contacted with the sample under investigation and any phosphatase-targeting toxin present in the sample binds to the immobilised phosphatase. A source of reporter molecules which compete with the toxin for phosphatase binding sites is added. The reporter molecules displace toxin molecules from the binding sites to a degree which depends upon the relative concentration of toxin molecules and reporter molecules. The degree of reporter molecule binding facilitates determination of toxin present in the sample under investigation. Preferred reporters/labels include radiolabels, chromophores (including fluorophores) and enzymes which give rise to chromogenic or fluorogenic products. Scintillation proximity labels and labels which give rise to a measurable change in light scattering are also to be considered.

In an alternative embodiment, solid support immobilised reporter-blocked phosphatase molecules are contacted with the sample under investigation and any phosphatase-targeting toxins present in the sample compete with the phosphatase bound reporter molecules displacing them from the solid phase into the aqueous phase in a degree proportional to the amount of toxin present in the sample. The amount of reporter molecule which remains bound to the solid phase is then assessed to facilitate determination of toxin presence in the sample under investigation.

Viewed from a further aspect, the invention provides a kit for the detection of cyanobacterial or algal phosphatase-targeting toxins, according to the invention, said kit comprising:
a solid phase upon which is immobilised a ligand;
a non-immobilized ligand, preferably in aqueous solution or complexed to the immobilized ligand; where neither of said immobilized and non-immobilized ligands includes a directly or indirectly detectable moiety, a reporter moiety capable of binding to one of said immobilized and non-immobilized ligands and generating a detectable signal, preferably said detectable moiety or signal being directly readable without laboratory equipment.

In one preferred embodiment, the kit of the present invention comprises:
a solid phase upon which is immobilized phosphatase-targeting toxin binding ligands;
a reporter molecule capable of competitively inhibiting binding of phosphatase-targeting toxins to said toxin binding ligand and generating a signal readable without laboratory equipment.

An especially preferred embodiment of the kit of the invention comprises magnetically displaceable polymer micro spheres having immobilized thereon a protein phosphatase;
gold sol labelled peptide hepatotoxin molecules capable of competitively inhibiting cyanobacterial toxins binding to said protein phosphatase.

A further especially preferred embodiment of the kit of the invention comprises magnetically displaceable polymer micro spheres having immobilized thereon a protein phosphatase;
gold sol labelled okadaic acid molecules capable of competitively inhibiting algal toxins binding to said protein phosphatase.

In another preferred aspect, use of the kit involves dipping a porous cellulosic substrate on which a toxin binding ligand is immobilized and which is impregnated with a competitively binding, chromophore (or fluorophore etc) labelled ligand into a sample of water or shellfish fluid, allowing the saturated substrate to incubate for a pre-set period (either removed from the sample or in a pre-set volume of the sample), removing non-bound labelled ligand, e.g. by flushing the substrate with toxin-free water or by leaving the substrate to soak for a pre-set period in a pre-set volume of toxin free water, and inspecting the colour of the substrate or of the soaking water. Desirably, the substrate is mounted on a support, preferably one marked with calibration colours to facilitate comparison of the substrate or soaking water colour to determine toxin concentration or to indicate whether toxin concentration is above or below one or more threshold values.

The invention will now be illustrated by the following non-limiting examples:

### Materials

Microcystin YR, Microcystin-LR, okadaic acid, nodularin, calyculin A and tautomycin are purchased from Calbiochem (San Diego, CA). Carrier-free Na¹²⁵I and [γ-³²P]ATP is obtained from Amersham (Little Chalfont, UK). Albumin (RIA grade), ammonium acetate, Chloramine T, dimethyl sulfoxide (DMSO), dithioerythritol (DTE), EDTA, EGTA, glycerol, Hepes, histone II-AS, sodium metabisulfite and trypsin inhibitor (soybean) are purchased from Sigma (St Louis, MO). Acetonitrile and trifluoroacetic acid (TFA) are purchased from Rathburn (Walkerburn, Scotland). Partially purified protein phosphatase 2A is either purchased from Upstate Biotechnology (Lake Placid, NY) or purified according to Resink et al. (Eur. J. Biochem. 133: 455-461 (1983)).

### Iodination of mycrocystin-YR

Microcystin YR (10 µg) is iodinated with 1 mCi carrier-free Na¹²⁵I (37 MBq) using chloramine T as described by Ciechanover et al., (PNAS 77: 1365-1368 (1980)). Following the iodination reaction, iodide is separated from [¹²⁵I]microcystin-YR using Sep-Pak® Plus cartridges (Waters, Milford, MA) according to the method of Runnegar et al. (Toxicon 24: 506-509 (1986)). The [¹²⁵I]microcystin-YR is applied to a 3x250 mm Inertsil ODS-2 HPLC column from Chrompack (Raritan, NJ) and eluted with an acetonitrile gradient.

### Competitive binding assay

The competitive binding assay is carried out in a volume of 0.5 ml buffered with 50 mM Hepes (pH 7.2), 1 mM EDTA, 0.3 mM EGTA, 1 mM DTE, 5 mM MnCl₂, 0.5 mg ml⁻¹ BSA, and 0.2 mg ml⁻¹ trypsin inhibitor. Algal toxins diluted in 100% DMSO are added to the assay at 0-100 nM in a final concentration of 10% DMSO. [¹²⁵I]microcystin-YR (1 Ci/13 ng) is added at 35 pM. Protein phosphatase 2A (30 pM) is added last, and the reaction mixture is incubated on ice overnight. [¹²⁵I]microcystin-YR bound to protein phosphatase 2A is separated from free [¹²⁵I]microcystin-YR by gel filtration using Sephadex® G-50 fine from Pharmacia (Uppsala, Sweden) in 0.7 x 15 cm columns from Bio-Rad (Hercules, CA). A 50 mM Hepes buffer (pH 7.2) with 1 mM EDTA and 0.3 mM EGTA is used in the separation which is done at 4°C. The fraction containing [¹²⁵I]microcystin-YR which binds to protein phosphatase 2A is collected and the radioactivity is quantitated by scintillation counting. Nonspecific binding of [¹²⁵I]microcystin-YR is detected in a control reaction where microcystin-LR is added at an excess (1 µM).

### Example 1

Protein phosphatase 2A is coupled to magnetic beads (directly to the beads or via biotinylation of the phosphatase). Immobilized protein phosphatase is then mixed with sample and radiolabelled toxin (e.g. [¹²⁵I]-microcystin-YR). The immobilized protein phosphatase is separated from the reaction mixture by magnetic force. Radioactivity associated with the protein phosphatase (magnetic bead) is detected by scintillation counting. The amount of radiolabel associated with the protein phosphatase decreases as a function of phosphatase binding toxin in the sample.

### Example 2

Protein phosphatase 2A is coupled to magnetic beads (directly to the beads or via biotinylation of the phosphatase). Immobilized protein phosphatase is then mixed with sample and toxin coupled to colored beads. The immobilized protein phosphatase is separated from the reaction mixture by magnetic force. Colored beads associated with the protein phosphatase (magnetic beads) are evaluated by eye or by a low magnification microscope (e.g. Nikon TMS). The amount of colored beads associated with the protein phosphatase (magnetic beads) decreases as a function of phosphatase binding toxin in the sample.

### Example 3

Protein phosphatase 2A is coupled to magnetic beads (directly to the beads or via biotinylation of the phosphatase). Immobilized protein phosphatase is then mixed with sample and toxin immobilized on beads carrying an immobilized enzyme. The enzyme is capable of producing a detectable product (colored or fluorescent) upon appropriate incubation with a chromogenic or fluorogenic substrate. The immobilized protein phosphatase is separated from the reaction mixture by magnetic force. Color or fluorescence associated with the protein phosphatase (magnetic beads) is measured by spectroscopy or fluorimetry, respectively. The amount of color/fluorescence associated with the magnetic beads decreases as a function of phosphatase binding toxin in the sample.

### Example 4

### Scintillation Proximity Assay:

Protein phosphatase is biotinylated and immobilized to wells precoated with streptavidin and a scintillant (e.g. FlashPlate PLUS Streptavidin SMP103 supplied by NEN). The sample and [¹²⁵I]microcystin-YR are added to the wells. The amount of [¹²⁵I]microcystin-YR bound to the immobilized protein phosphatase is detected by scintillation counting.

### Example 5

| Inhibition of binding of [¹²⁵I]-microcystin-YR to protein phosphatase 2A in the presence of various toxins | |
|---|---|
| Compound tested¹ | IC₅₀² (pM) |
| nodularin | 15 |
| microcystin-LR | 17 |
| microcystin-YR | 75 |
| okadaic acid | 100 |
| calyculin A | 251 |
| tautomycin | 562 |

| | |
|---|---|
| ¹ The compounds tested were incubated with [¹²⁵I]-microcystin-YR and protein phosphatase 2A as described above. | |
| ² The IC₅₀ value represents the concentration needed to obtain a 50% inhibition of [¹²⁵I]-microcystin-YR binding to protein phosphatase 2A. These values were determined according to Fig. 3. The data represent an average of at least 3 separate experiments. | |

### Example 6

| Effect of exogenous compounds on the competitive binding assay as compared to the protein phosphatase assay | | |
|---|---|---|
| Compound tested¹ | % activity² | |
| | Competitive binding assay | Protein phosphatase assay |
| 2 mM ATP | 103.3 ± 0.2 | 9.8 ± 3.4 |
| 0.5 mM ATP | 101.6 ± 1.7 | 29.8 ± 5.6 |
| 0.05 mM NaPPi | 101.4 ± 4.1 | 14.2 ± 1.2 |
| 50 mM NaF | 101.5 ± 1.9 | 7.7 ± 1.4 |
| 5 mM NaF | 102.0 ± 3.3 | 62.6 ± 0.4 |
| 1 mg/ml caseine | 98.6 ± 4.5 | 3.4 ± 0.2 |
| 0.02 mg/ml caseine | 98.9 ± 6.1 | 33.3 ± 4.9 |
| 5 mg/ml histone 2A | 91.9 ± 1.8 | 1.4 ± 0.1 |
| 0.002 mg/ml histone | 95.2 ± 4.7 | 63.6 ± 4.0 |
| 0.5 M NaCl | 41.2 ± 0.7 | 44.4 ± 1.6 |
| seawater | 34.8 ± 0.4 | ND |
| 10% seawater | 87.3 ± 0.4 | ND |
| 10% DMSO | 72.8 ± 2.3 | 97.9 ± 3.3 |
| 10% MeOH | 73.9 ± 0.5 | 87.4 ± 4.1 |
| 10% acetonitrile | 90.4 ± 5.4 | 88.2 ± 2.7 |
| 0.4% Triton X-100 | 122.3 ± 1.0 | 60.2 ± 5.7 |
| 0.4% Nonidet P-40 | 106.0 ± 2.0 | 61.1 ± 1.3 |
| 0.4% CHAPS | 90.9 ± 9.9 | 138.0 ± 34.4 |

| | | |
|---|---|---|
| ¹ Protein phosphatase 2A was preincubated with the compounds dissolved in 50 mM Hepes (pH 7.2) or with buffer alone (control) for 30 minutes on ice. Phosphatase activity was measured by dephosphorylation of phosphohistone as described. The % activity is relative to the control reaction. | | |
| ² The activity in the competitive binding assay represents the ability of protein phosphatase 2A to bind [¹²⁵I]microcystin-YR in the presence of the exogenous compound dissolved in buffer relative to buffer alone. The data represents an average of at least three separate experiments ± SEM. | | |

### Example 7

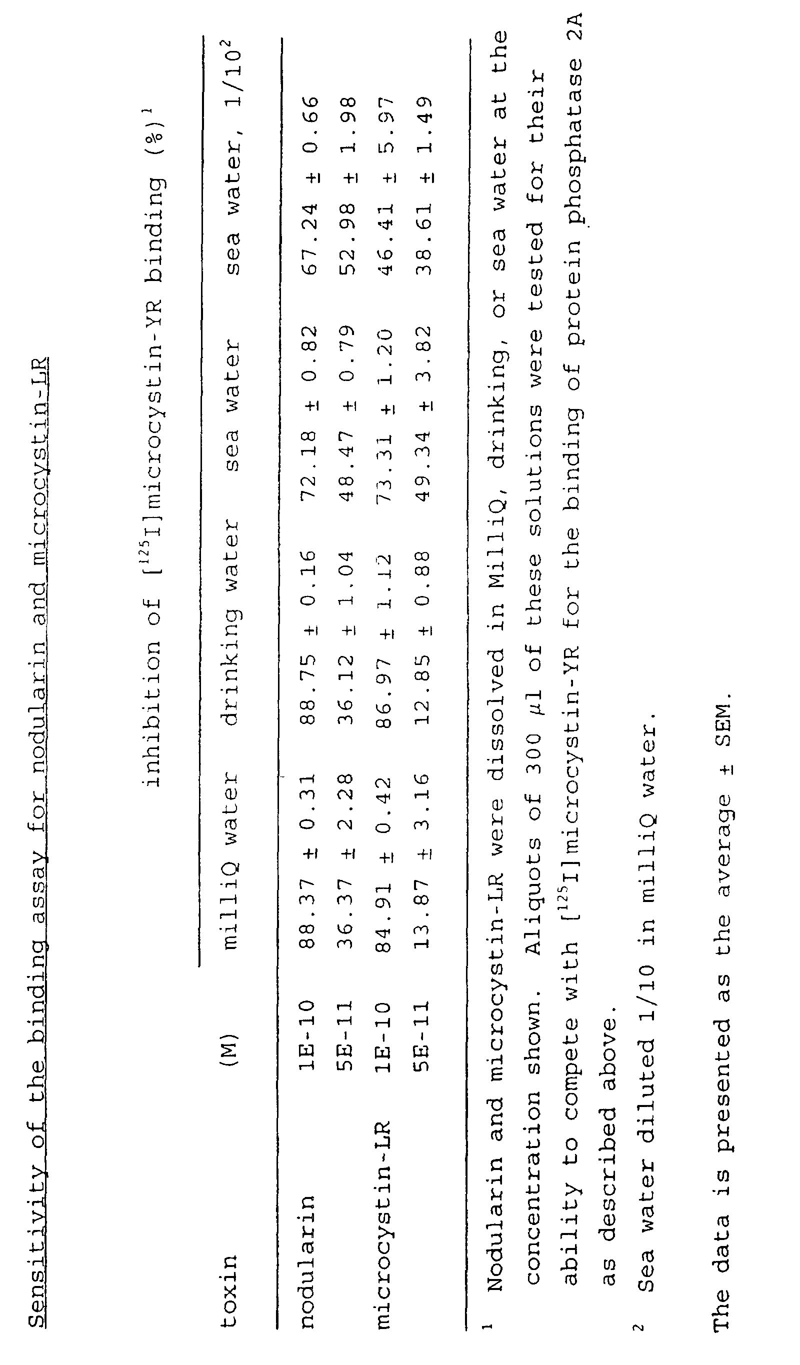

### Example 9

| Okadaic acid equivalents in shellfish extracts as determined by HPLC analysis and by the protein phosphatase binding assay | | | |
|---|---|---|---|
| Extract¹ | OA equivs. by HPLC analysis² | OA equivs by binding assay³ | |
| | (µg/g hepatopancreas) | (nM) | (nM) |
| 1 | 0 | 0 | 85 |
| 2 | 0 | 0 | 45 |
| 3 | 0 | 0 | 70 |
| 4 | 4 | 2480 | 2100 |
| 5 | 1.2 | 748 | 755 |
| 6 | 0.8 | 496 | 805 |

| | | | |
|---|---|---|---|
| ¹ The extracts were made from hepatopancreas of mussels collected along the Norwegian coast. | | | |
| ² The extracts were analyzed for okadaic acid equivalents by HPLC. | | | |
| ³ The extracts were diluted in 100% DMSO and tested for their ability to compete with [¹²⁵I]microcystin-YR for binding to protein phosphatase 2A using the binding assay as described above. The concentration of okadaic acid equivalents were determined by comparing the data to standard curves of okadaic acid dissolved in 100% DMSO. | | | |

### Example 9

### Attached Diagrams

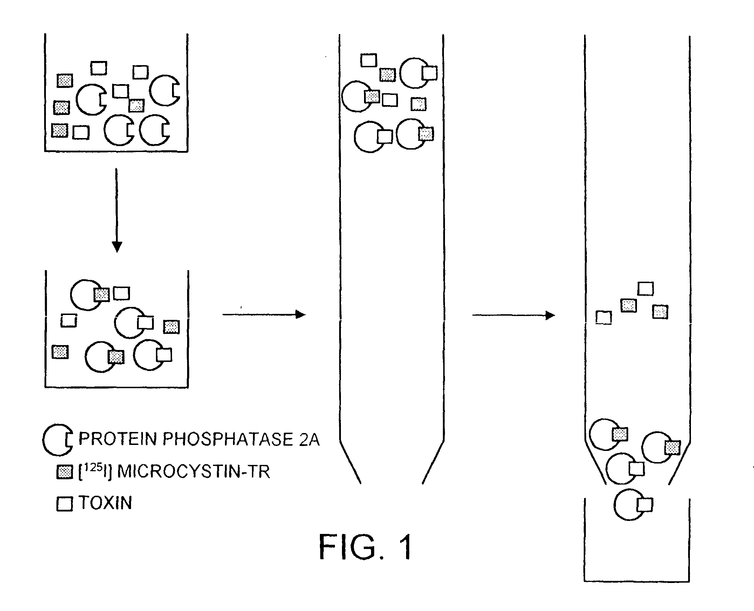

Fig. 1 of the attached diagram is a schematic diagram of the competitive binding assay for the detection of protein phosphatase binding toxins.

Protein phosphatase 2A is incubated with [¹²⁵I]microcystin-YR and another toxin directed towards protein phosphatase 2A. The toxin competes with the [¹²⁵I]microcystin-YR for binding to the phosphatase. Addition of a large amount of toxin results in a reduced binding of [¹²⁵I]microcystin-YR to the phosphatase and vice versa. After binding equilibrium is reached, the [¹²⁵I]microcystin-YR bound to protein phosphatase 2A is separated from free [¹²⁵I]microcystin-YR by gel filtration chromatography. The fraction containing [¹²⁵I]microcystin-YR bound to the phosphatase is collected and the amount of radioactivity determined by scintillation counting.

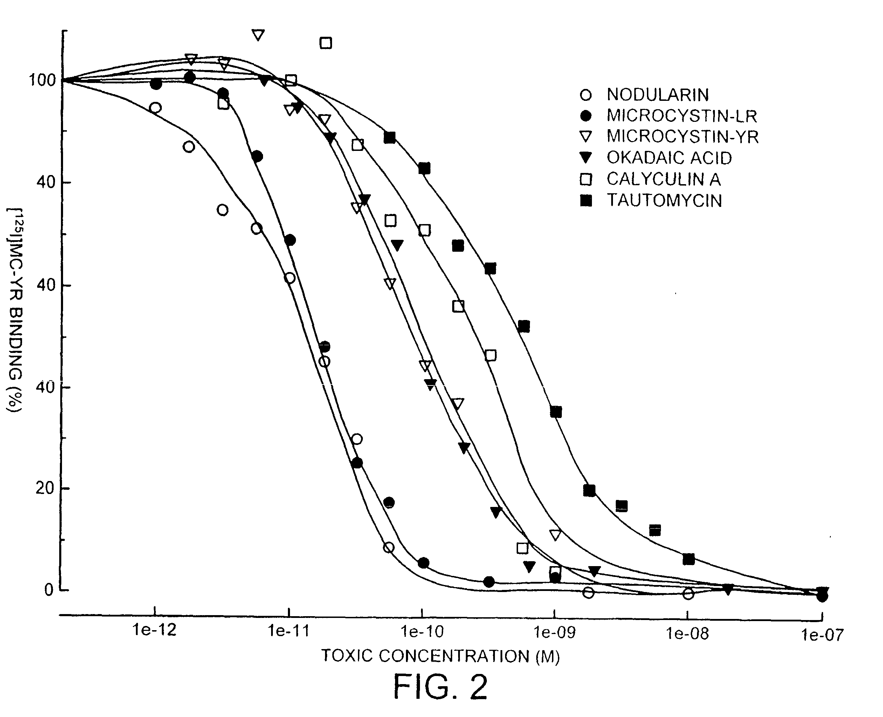

Fig. 2 of the attached diagrams shows the effect of increasing amounts of different algal toxins on binding of [¹²⁵I]microcystin-YR to protein phosphatase 2A.

Protein phosphatase 2A (30 pM) was incubated in the presence 35pM [¹²⁵I]microcystin-YR (l Ci/13 ng) and 0-100 nM of different algal toxins indicated in the figure. The [¹²⁵I]microcystin-YR bound to protein phosphatase 2A was isolated by gel filtration chromatography and the radioactivity determined by scintillation counting. Each curve represents the average of at least 3 separate experiments.

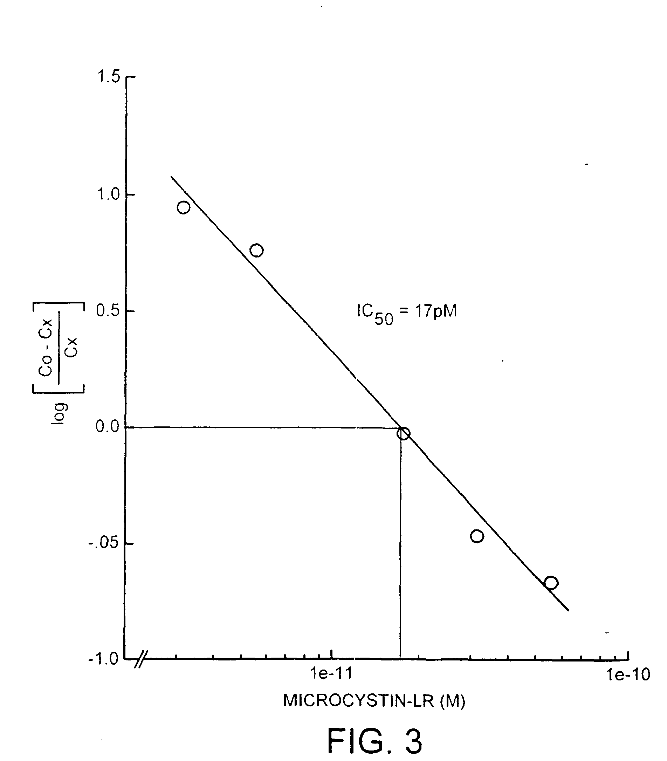

Fig. 3 of the attached diagrams shows the IC₅₀ for microcystin-LR binding in the competitive binding assay.

Binding of [¹²⁵I]microcystin-YR to protein phosphatase 2A was plotted as the ratio between unbound [¹²⁵I]microcystin-YR (Co-Cx) and bound [¹²⁵I]microcystin-YR (Cx) against the concentration of microcystin-LR. Co represents the amount of bound [¹²⁵I]microcystin-YR in the absence of microcystin-YR, and Cx represents the amount of bound [¹²⁵I]microcystin-YR in the presence of various concentrations of microcystin-LR.

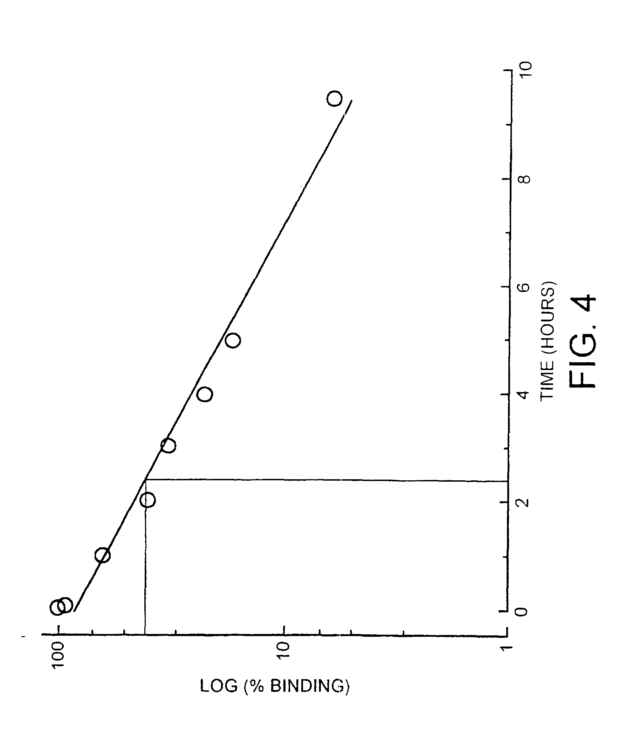

Fig. 4 of the attached diagrams illustrates the stability of the [¹²⁵I]microcystin-YR bound to protein phosphatase 2A in the presence of excess microcystin LR.

Protein phosphatase 2A (1 nM) was incubated in the presence of [¹²⁵I]microcystin-YR (100 pM) for 1 hour. Microcystin-LR (2 µM) was added to the reaction mixture at time 0. The amount of [¹²⁵I]microcystin-YR bound to protein phosphatase 2A was determined for the indicated timepoints by gel filtration and scintillation counting as described. The curve represents an average of 4 separate experiments.

## Claims

1. An assay method for determining phosphatase targeting toxins which inhibit protein phosphatases comprising contacting a solid support having an immobilized ligand immobilized thereon with:
(i) a sample suspected of being contaminated with toxin and
(ii) a non-immobilized ligand,
wherein said immobilized ligand is capable of binding to at least one of said toxins, to said non-immobilized ligand or to complexes of said toxin and said non-immobilized ligand, and said non-immobilized ligand is capable of binding to at least one of said immobilized ligand, to said toxin or to complexes of said toxin and said immobilized ligand whereby the proportion of said immobilized ligand bound by said toxin, said non-immobilized ligand or complexes of said toxin and said non-immobilized ligand is dependent on the toxin content of said sample and wherein the immobilized and/or non-immobilized toxin binding ligand is a protein phosphatase enzyme and
wherein said immobilized ligand is capable of generating directly or indirectly detectable signal when uncomplexed, when complexed by said toxin, when complexed by a complex of said toxin and said non-immobilized ligand or when complexed by said non-immobilized ligand or said non-immobilized ligand is capable of generating a directly or indirectly detectable signal when uncomplexed or when complexed,
separating a bound fraction from a non-bound fraction; and
directly or indirectly determining the non-immobilized ligand bound to the immobilized ligand (the bound fraction) or non-complexed in aqueous solution (the non-bound fraction);
wherein the application of (i) and (ii) to the solid support may be performed separately, sequentially or simultaneously and if separately or sequentially, they can be performed in either order.

2. An assay method as claimed in claim 1 wherein said phosphatase-targeting toxin is produced by algae or by cyanobacteria.

3. An assay method as claimed in either of claims 1 and 2 wherein the toxin to be determined is a hepatotoxin or okadaic acid.

4. An assay method as claimed in any one of claims 1 to 3 wherein toxin molecules present in the sample compete with the non-immobilized ligand for a limited number of binding sites of the immobilized ligand and any toxin present in said sample is determined relative to the extent of non-immobilized ligand bound to or not bound to the binding sites of the immobilized ligand.

5. An assay method as claimed in any one of claims 1 to 4 wherein the presence or absence of a phosphatase-targeting toxin is determined.

6. An assay method as claimed in any one of claims 1 to 5 wherein the sample under investigation is surface or free moisture from shellfish, or water taken from the habitat in which such shellfish live, or water taken from domestic water supplies.

7. An assay method as claimed in any one of claims 1 to 6 wherein the immobilized or non-immobilized ligand is an antibody or antibody fragment.

8. An assay method as claimed in any one of claims 1 to 7 wherein the protein phosphatase enzyme is the binding ligand protein phosphatase 2A.

9. An assay method as claimed in any one of claims 1 to 8 wherein either the immobilised or non-immobilised ligands carries a reporter moiety.

10. An assay method as claimed in claim 9 wherein the non-immobilized ligand carries a reporter moiety.

11. An assay method as claimed in claim 10 wherein the non-immobilized ligand is a labelled peptide hepatotoxin or labelled okadaic acid.

12. An assay method as claimed in claim 11 wherein the hepatotoxin is selected from nodularin, microcystin LR or microcystin YR.

13. An assay method as claimed in any one of claims 1 to 12 wherein the solid support is a dipstick or solid matrix.

14. An assay method as claimed in claim 13 wherein the solid matrix is polymeric or magnetic beads.

15. A kit for the detection of phosphatase-targeting toxins which inhibit protein phosphatases, said kit comprising:
a solid phase upon which is immobilised a ligand;
a non-immobilized ligand, preferably in aqueous solution or complexed to the immobilized ligand;
and wherein the immobilized and/or non-immobilized ligand is a protein phosphatase enzyme;
where neither of said immobilized and non-immobilized ligands includes a directly or indirectly detectable moiety, a reporter moiety capable of binding to one of said immobilized and non-immobilized ligands and generating a detectable signal, preferably said detectable moiety or signal being directly readable without laboratory equipment.

16. A kit as claimed in claim 15 wherein said phosphatase-targeting toxin is produced by algae or by cyanobacteria.

17. A kit for the detection of phosphatase-targeting toxins which inhibit protein phosphatases, wherein said kit comprises:
a solid phase upon which is immobilized a protein phosphatase enzyme as a toxin binding ligand;
a reporter molecule capable of competitively inhibiting binding of phosphatase-targetting toxins to said toxin binding ligand and generating a signal readable without laboratory equipment.

18. A kit as claimed in any one of claims 15 to 17 wherein said kit comprises magnetically displaceable polymer micro spheres having immobilized thereon a protein phosphatase;
gold sol labelled peptide hepatotoxin molecules capable of competitively inhibiting cyanobacterial toxins binding to said protein phosphatase.

19. A kit as claimed in any one of claims 15 to 17 wherein said kit comprises magnetically displaceable polymer micro spheres having immobilized thereon a protein phosphatase;
gold sol labelled okadaic acid molecules capable of competitively inhibiting algal toxins binding to said protein phosphatase.

20. A kit for the detection of phosphatase-targeting toxins which inhibit protein phosphatases, said kit comprising:
a solid phase upon which is immobilized a ligand;
a non-immobilized ligand, preferably in aqueous solution or complexed to the immobilized ligand;
and wherein the immobilized and/or non-immobilized ligand is a protein phosphatase enzyme;
where either of said immobilized and non-immobilized ligands carries a reporter moiety capable of generating a detectable signal, preferably said detectable signal being directly readable without laboratory equipment.

21. Use of the kit as claimed in any one of claims 15 to 20 for the determination of phosphatase-targeting toxins.

## Patentansprüche

1. Testmethode zur Bestimmung von auf Phosphatase zielenden Toxinen, welche Proteinphosphatasen inhibieren, wobei man einen festen Träger, der einen daran immobilisierten Liganden aufweist, in Kontakt bringt mit:
(i) einer Probe von der man annimmt, dass sie mit Toxin kontaminiert ist und
(ii) einem nicht-immobilisierten Liganden,
wobei der immobilisierte Ligand befähigt ist zur Bindung an wenigstens eines der Toxine, an den nicht-immobilisierten Liganden oder an Komplexe des Toxins und des nicht-immobilisierten Liganden, und
wobei der nicht-immobilisierte Ligand befähigt ist zur Bindung an wenigstens einen der immobitisierten Liganden, an das Toxin oder an Komplexe aus dem Toxin und dem immobilisierten Liganden, wobei der Anteil des immobilisierten Liganden, gebunden von dem Toxin, von dem nicht-immobilisierten Liganden oder Komplexen des Toxins und des nicht-immobilisierten Liganden abhängig ist von Toxingehalt der Probe und worin der immobilisierten Liganden und/oder nicht-immobilisierte Toxin-bindende Ligand ein Proteinphosphatase-Enzym ist und
worin der immobilisierte Ligand befähigt ist, ein direkt oder indirekt nachweisbares Signal zu erzeugen, wenn er nicht komplexiert ist, wenn er mit dem Toxin komplexiert ist, wenn er mit einem Komplex aus dem Toxin und dem nicht-immobilisierten Liganden komplexiert ist, oder wenn er mit dem nicht-immobilisierten Liganden komplexiert ist; oder der nicht-immobilisierte Ligand befähigt ist, ein direkt oder indirekt nachweisbares Signal zu erzeugen, wenn er unkomplexiert oder komplexiert vorliegt;
eine gebundene Fraktion von einer nicht-gebundenen Fraktion trennt; und
direkt oder indirekt den nicht-immobilisierten Liganden, gebunden an den immobilisierten Liganden (die gebundene Fraktion) oder in nichtkomplexierter Form in wässriger Lösung (die nicht-gebundene Fraktion) bestimmt;
wobei die Anwendung von (i) und (ii) auf den festen Träger voneinander getrennt, nacheinander oder gleichzeitig und bei getrennter oder sequenzieller Anwendung, in beliebiger Reihenfolge durchführbar ist.

2. Testmethode nach Anspruch 1, worin das auf Phosphatase zielende Toxin von Algen oder Cyanobakterien produziert wird.

3. Testmethode nach einem der Ansprüche 1 und 2, worin das zu bestimmende Toxin ein Hepatotoxin oder Okadainsäure ist.

4. Testmethode nach einem der Ansprüche 1 bis 3, worin die in der Probe vorliegenden Toxinmoleküle mit dem nicht-immobilisierten Liganden um eine limitierte Anzahl von Bindungsstellen auf dem immobilisierten Liganden kompetitieren und jegliches in der Probe vorliegende Toxin relativ zu dem Maß an nicht-immobilisierten Liganden bestimmt wird, der an die Bindungsstellen des immobilisierten Liganden gebunden oder nicht gebunden ist.

5. Testmethode nach einem der Ansprüche 1 bis 4, worin das Vorliegen oder das Fehlen eines auf Phosphatase zielenden Toxins bestimmt wird.

6. Testmethode nach einem der Ansprüche 1 bis 5, worin die untersuchte Probe die Oberfläche oder die freie Flüssigkeit eines Schalentiers, oder Wasser aus dem Habitat, in welchem ein solches Schalentier lebt, oder Wasser aus der Hauswasserversorgung ist.

7. Testmethode nach einem der Ansprüche 1 bis 6, worin der immobilisierte oder nicht-immobilisierte Ligand ein Antikörper oder Antikörperfragment ist.

8. Testmethode nach einem der Ansprüche 1 bis 7, worin das Protein Phosphatase-Enzym der Proteinphosphatase 2A-Bindungsligand ist.

9. Testmethode nach einem der Ansprüche 1 bis 8, worin entweder der immobilisierte oder der nicht-immobilisierte Ligand einen Reporter-Rest trägt.

10. Testmethode nach Anspruch 9, worin der nicht-immobilisierte Ligand einen Reporter-Rest trägt.

11. Testmethode nach Anspruch 10, worin der nicht-immobilisierte Ligand ein markiertes Peptid Heptatoxin oder markierte Okadainsäure ist.

12. Testmethode nach Anspruch 11, worin das Hepatotoxin ausgewählt ist unter Nodularin, Microcystin LR oder Microcystin YR.

13. Testmethode nach einem der Ansprüche 1 bis 12, worin der feste Träger ein Messstäbchen oder eine feste Matrix ist.

14. Testmethode nach Anspruch 13, worin als feste Matrix Polymerkügelchen oder Magnetkügelchen verwendet werden.

15. Kit zur Detektion von auf Phosphatase zielenden Toxinen, welche Proteinphosphatasen inhibieren, wobei der Kit umfasst:
eine feste Phase, auf welcher ein Ligand immobilisiert ist;
einen nicht-immobilisierten Liganden, vorzugsweise in wässriger Lösung oder komplexiert mit dem immobilisierten Liganden;
und worin der immobilisierte und/oder nicht-immobilisierte Ligand ein Proteinphosphatase-Enzym ist;
wobei weder der immobilisierte noch der nicht-immobilisierte Ligand einen direkt oder indirekt detektierbaren Rest enthält; oder
einen Reporter-Rest, der zur Bindung an einen der immobilisierten und nicht-immobilisierten Liganden und zur Erzeugung eines detektierbaren Signals befähigt ist, wobei vorzugsweise der detektierbare Rest oder das detektierbare Signal ohne Laborausrüstung ablesbar ist.

16. Kit nach Anspruch 15, wobei das auf Phosphatase zielende Toxin von Algen oder Cyanobakterien produziert wird.

17. Kit zum Nachweis eines auf Phosphatase zielenden Toxins, welches Proteinphosphatasen inhibiert, wobei der Kit umfasst:
einen festen Träger, auf welchen ein Proteinphosphatase-Enzym als Toxin-bindender Ligand immobilisiert ist; und
ein Reporter-Molekül, welches zur kompetitiven Inhibition des Bindung von auf Phosphatase zielenden Toxinen an den Toxin-bindenden Liganden und zur Erzeugung eines ohne Laborausrüstung ablesbaren Signals befähigt ist.

18. Kit nach einem der Ansprüche 15 bis 17, wobei der Kit magnetisch entfernbare polymere Mikrokügelchen umfasst, worauf eine Proteinphosphatase immobilisiert ist; und
Goldsol-markierte Peptidhepatotoxin-Moleküle, welche zur kompetitiven Inhibierung der Bindung von cyanobakteriellen Toxinen an die Proteinphosphatase befähigt sind.

19. Kit nach einem der Ansprüche 15 bis 17, wobei der Kit magnetisch entfernbare polymere Mikrokügelchen umfasst, auf denen eine Proteinphosphatase immobilisiert ist; und
Goldsol-markierte Okadainsäuremoleküle, welche zur kompetitiven Inhibierung der Bindung von Algentoxinen an die Proteinphosphatase befähigt sind.

20. Kit zur Detektion von auf Phosphatase zielenden Toxinen, welche Proteinphosphatasen inhibieren, wobei der Kit umfasst:
eine feste Phase, auf welcher ein Ligand immobilisiert ist;
einen nicht-immobilisierten Liganden, vorzugsweise in wässriger Lösung oder komplexiert mit dem immobilisierten Liganden;
und worin der immobilisierte und/oder nicht-immobilisierte Ligand ein Proteinphosphatase-Enzym ist;
wobei einer der immobilisierten und nicht-immobilisierten Liganden einen Reporter-Rest trägt, der zur Erzeugung eines detektierbaren Signals befähigt ist, wobei vorzugsweise das detektierbare Signal ohne Laborausrüstung ablesbar ist.

21. Verwendung eines Kits nach einem der Ansprüche 15 bis 20 zur Bestimmung von auf Phosphatase zielenden Toxinen.

## Revendications

1. Procédé de dosage pour déterminer des toxines de ciblage de phosphatases qui inhibent les protéines phosphatases, comprenant le fait de mettre en contact un support solide, sur lequel un ligand immobilisé est immobilisé, avec :
(i) un échantillon soupçonné d'être contaminé par une toxine et
(ii) un ligand non immobilisé,
dans lequel ledit ligand immobilisé peut se lier à au moins l'une desdites toxines, audit ligand non immobilisé, ou aux complexes de ladite toxine et dudit ligand non immobilisé, et ledit ligand non immobilisé peut se lier à au moins l'un desdits ligands immobilisés, à ladite toxine, ou aux complexes de ladite toxine et dudit ligand immobilisé, moyennant quoi la proportion dudit ligand immobilisé lié par ladite toxine, dudit ligand non immobilisé ou des complexes de ladite toxine et dudit ligand non immobilisé, dépend de la teneur en toxine dudit échantillon, et dans lequel le ligand, liant la toxine, immobilisé et/ou non immobilisé, est une enzyme protéine phosphatase, et
dans lequel ledit ligand immobilisé peut générer un signal détectable directement ou indirectement lorsqu'il est non complexé, lorsqu'il est complexé par ladite toxine, lorsqu'il est complexé par un complexe de ladite toxine et dudit ligand non immobilisé ou lorsqu'il est complexé par ledit ligand non immobilisé, ou ledit ligand non immobilisé peut générer un signal détectable directement ou indirectement lorsqu'il est non complexé ou lorsqu'il est complexé,
séparer une fraction liée d'une fraction non liée ; et
déterminer, directement ou indirectement, le ligand non immobilisé lié au ligand immobilisé (fraction liée), ou non complexé en solution aqueuse (fraction non liée) ;
dans lequel les applications de (i) et de (ii) au support solide peuvent être effectuées de façon séparée, séquentielle ou simultanée, et dans les deux premiers cas, elles peuvent être effectuées dans n'importe quel ordre.

2. Procédé de dosage selon la revendication 1, dans lequel ladite toxine de ciblage de phosphatases est produite par des algues ou par des cyanobactéries.

3. Procédé de dosage selon l'une ou l'autre des revendications 1 et 2, dans lequel la toxine à déterminer est une hépatotoxine ou l'acide okadaïque.

4. Procédé de dosage selon l'une quelconque des revendications 1 à 3, dans lequel les molécules de toxine présentes dans l'échantillon rivalisent avec le ligand non immobilisé pour un nombre limité de sites de fixation du ligand immobilisé, et toute toxine présente dans ledit échantillon est déterminée par rapport à l'étendue du ligand non immobilisé lié ou non lié aux sites de fixation du ligand immobilisé.

5. Procédé de dosage selon l'une quelconque des revendications 1 à 4, dans lequel on détermine la présence ou l'absence d'une toxine de ciblage de phosphatases.

6. Procédé de dosage selon l'une quelconque des revendications 1 à 5, dans lequel l'échantillon à l'étude est l'eau de surface ou libre des mollusques et des crustacés, ou l'eau prélevée de l'habitat dans lequel de tels mollusques et crustacés vivent, ou l'eau prélevée des canalisations de distribution d'eau.

7. Procédé de dosage selon l'une quelconque des revendications 1 à 6, dans lequel le ligand immobilisé ou non immobilisé est un anticorps ou un fragment d'anticorps.

8. Procédé de dosage selon l'une quelconque des revendications 1 à 7, dans lequel l'enzyme protéine phosphatase est la protéine phosphatase 2A du ligand de liaison.

9. Procédé de dosage selon l'une quelconque des revendications 1 à 8, dans lequel le ligand immobilisé ou le ligand non immobilisé porte un fragment de marquage.

10. Procédé de dosage selon la revendication 9, dans lequel le ligand non immobilisé porte un fragment de marquage.

11. Procédé de dosage selon la revendication 10, dans lequel le ligand non immobilisé est une hépatotoxine peptidique marquée ou l'acide okadaïque marqué.

12. Procédé de dosage selon la revendication 11, dans lequel l'hépatotoxine est choisie parmi la nodularine, la microcystine LR ou la microcystine YR.

13. Procédé de dosage selon l'une quelconque des revendications 1 à 12, dans lequel le support solide est une bandelette ou une matrice solide.

14. Procédé de dosage selon la revendication 13, dans lequel des perles polymères ou magnétiques constituent la matrice solide.

15. Trousse de détection des toxines de ciblage de phosphatases qui inhibent les protéines phosphatases, ladite trousse comprenant :
une phase solide sur laquelle un ligand est immobilisé ;
un ligand non immobilisé, de préférence en solution aqueuse ou complexé au ligand immobilisé ;
et dans laquelle le ligand immobilisé et/ou le ligand non immobilisé est une enzyme protéine phosphatase ;
où ni l'un ni l'autre desdits ligands immobilisés et non immobilisés comprennent un fragment directement ou indirectement détectable, un fragment de marquage pouvant se lier à l'un desdits ligands immobilisés et non immobilisés et générer un signal détectable, de préférence, ledit fragment détectable ou ledit signal détectable étant directement lisible sans équipement de laboratoire.

16. Trousse selon la revendication 15, dans laquelle ladite toxine de ciblage de phosphatases est produite par des algues ou par des cyanobactéries.

17. Trousse de détection des toxines de ciblage de phosphatases qui inhibent les protéines phosphatases, dans laquelle ladite trousse comprend :
une phase solide sur laquelle une enzyme protéine phosphatase, en tant que ligand liant les toxines, est immobilisée ;
une molécule de marquage pouvant inhiber, de façon compétitive, la liaison des toxines de ciblage de phosphatases au ligand liant ladite toxine, et générer un signal lisible sans équipement de laboratoire.

18. Trousse selon l'une quelconque des revendications 15 à 17, dans laquelle ladite trousse comprend des microsphères de polymère pouvant être déplacées magnétiquement, une protéine phosphatase étant immobilisée sur celles-ci ;
des molécules d'hépatotoxine peptidique marquées par un sol d'or pouvant inhiber, de façon compétitive, les toxines cyanobactériennes se liant à ladite protéine phosphatase.

19. Trousse selon l'une quelconque des revendications 15 à 17, dans laquelle ladite trousse comprend des microsphères de polymère pouvant être déplacées magnétiquement, une protéine phosphatase étant immobilisée sur celles-ci ;
des molécules d'acide okadaïque marquées par un sol d'or pouvant inhiber, de façon compétitive, les toxines d'algues se liant à ladite protéine phosphatase.

20. Trousse de détection des toxines de ciblage de phosphatases qui inhibent les protéines phosphatases, ladite trousse comprenant :
une phase solide sur laquelle un ligand est immobilisé ;
un ligand non immobilisé, de préférence en solution aqueuse ou complexé au ligand immobilisé ;
et dans laquelle le ligand immobilisé et/ou le ligand non immobilisé est une enzyme protéine phosphatase ;
où l'un ou l'autre desdits ligands immobilisés et non immobilisés comprend un fragment de marquage pouvant générer un signal détectable, ledit signal détectable de préférence étant directement lisible sans équipement de laboratoire.

21. Utilisation de la trousse selon l'une quelconque des revendications 15 à 20, pour la détermination des toxines de ciblage de phosphatases.
